# FASCICULE DE BREVET EUROPEEN

(11) **EP 1 257 642 B1**
(45) Date de publication et mention de la délivrance du brevet: **26.11.2008**
(21) Numéro de dépôt: 01907727.0
(22) Date de dépôt: 07.02.2001
(51) Int. Cl.: C12N 15/12, C07K 14/47, C12Q 1/68, C12N 15/11, A61K 38/00

(54) **PARTENAIRES DU DOMAINE PTB1 DE FE65, PREPARATION ET UTILISATIONS**
PARTNERN DES PTB1 DOMÄNE AUS FE65, HERSTELLUNG UND VERWENDUNGEN DAVON
PARTNERS OF PTB1 DOMAIN OF FE65, PREPARATION AND USES

(30) Priorité: 10.02.2000 FR 0001628; 18.04.2000 US 198500 P
(43) Date de publication de la demande: 20.11.2002
(73) Titulaire: Aventis Pharma S.A., 92160 Antony (FR)
(72) Inventeur: MAURY, Isabelle, F-94400 Vitry sur Seine (FR); MERCKEN, Luc, F-94100 Saint Maur (FR); FOURNIER, Alain, F-92290 Chatenay Malabry (FR)
(74) Mandataire: Bouvet, Philippe
(86) Numéro de dépôt international: PCT/FR2001/000361
(87) Numéro de publication internationale: WO 2001/059104

(56) Documents cités:
- WO-A-99/26961
- S. PIÑOL-ROMA ET AL.: "A novel heterogenous nuclear RNP protein with a unique distribution on nascent trascripts." THE JOURNAL OF CELL BIOLOGY, vol. 109, décembre 1989 (1989-12), pages 2575-2587, XP000949252 cité dans la demande
- H.H. RASMUSSEN ET AL.: "Microsequences of 145 proteins recorded in the two-dimensional gel protein database of normal epidermal keratinocytes." ELECTROPHORESIS, vol. 13, 1992, pages 960-969, XP000949245
- ZAMBRANO N. ET AL: "The Fe65 adaptor protein interacts through its PID1 domain with the transcription factor CP2/LSF/LBP1." JOURNAL OF BIOLOGICAL CHEMISTRY, (7 AUG 1998) 273/32 (20128-20133)., XP000946307
- ZAMBRANO N. ET AL: "Interaction of the phosphotyrosine interaction/phosphotyrosine binding- related domains of Fe65 with wild-type and mutant Alzheimer's.beta.-amyloid precursor proteins." JOURNAL OF BIOLOGICAL CHEMISTRY, (1997) 272/10 (6399-6405)., XP000946308 cité dans la demande
- BORG J.-P. ET AL: "[ PTB, a protein-protein interaction domain important for signal transduction]. LE 'JEU DE DOMINOS' DE LA TRANSMISSION DU SIGNAL." MEDECINE/SCIENCES, (1997) 13/5 (647-656)., XP000949265
- MCLOUGHLIN D.M. ET AL: "The intracellular cytoplasmic domain of the Alzheimer's disease amyloid precursor protein interacts with phosphotyrosine-binding domain proteins in the yeast two-hybrid system." FEBS LETTERS, (1996) 397/2-3 (197-200)., XP000929647 cité dans la demande
- FIORE F. ET AL: "The regions of the Fe65 protein homologous to the phosphotyrosine interaction/phosphotyrosine binding domain of Shc bind th intracellular domain of the Alzheimer's amyloid precursor protein." JOURNAL OF BIOLOGICAL CHEMISTRY, (1995) 270/52 (30853-30856)., XP000946306 cité dans la demande
- BORG J.-P. ET AL: "The phosphotyrosine interaction domains of X11 and FE65 bind to distinct sites on the YENPTY motif of amyloid precursor protein." MOLECULAR AND CELLULAR BIOLOGY, (1996) 16/11 (6229-6241)., XP000946305 cité dans la demande

## Description

La maladie d'Alzheimer (MA) est une maladie neurodégénérative qui affecte une large proportion de la population âgée. Cette maladie est caractérisée sur le plan clinique par la perte des fonctions cognitives, et sur le plan neuropathologique par la présence dans le cerveau de dépôts neurofibrillaires intracellulaires et de dépôts extracellulaires du peptide β-amyloïde (Aβ) formant les plaques amyloïdes (Yankner, 1996). Les plaques amyloïdes sont majoritairement composées des peptides Aβ à 40 ou 42 acides aminés qui sont générés par un processus protéolytique de la protéine précurseur du peptide β-amyloïde (APP) (Golde *et al*., 1992). Les dépôts extracellulaires d'Aβ sont spécifiques de la MA. Ils représentent la caractéristique précoce et invariable de toutes les formes de MA, incluant les formes familiales. Les formes familiales de la maladie apparaissent de manière relativement précoce (entre 40 et 60 ans) et sont dues à des mutations dans le gène de l'APP et dans les gènes de la préséniline-1 (PS1) et de la préséniline-2 (PS2). Les mutations dans ces trois gènes induisent des changements dans la protéolyse de l'APP, conduisant à une surproduction d'Aβ et à l'apparition précoce de la pathologie et des symptômes qui sont similaires à ceux des formes sporadiques de la MA.

L'internalisation de l'APP membranaire est une étape nécessaire au processus de protéolyse de l'APP (Koo et Squazzo, 1994) qui dépend de son domaine cytoplasmique. En effet, la délétion de cette région de la protéine ou la présence de mutations ponctuelles au niveau de la séquence Tyr-Glu-Asn-Pro-Thr-Tyr dans le domaine cytoplasmique de l'APP, induit une diminution importante de la production du peptide β-amyloïde (Perez *et al*., 1999). Plusieurs protéines ont été identifiées comme interagissant avec le domaine cytoplasmique de l'APP, celles-ci pourraient donc participer à la régulation du processus protéolytique de l'APP et donc à la production du peptide β-amyloïde. Citons les deux familles de protéines FE65 et X11 qui interagissent avec la séquence Tyr-Glu-Asn-Pro-Thr-Tyr du domaine cytoplasmique de l'APP (Borg *et al*., 1996, 1998 ; Bressler *et al*., 1996 ; Duilio *et al*., 1998 ; Fiore *et al*., 1995 ; Guénette *et al*., 1996 ; McLoughlin et Miller, 1996 ; Mercken *et al*., 1998 ; Tanahashi et Tabira, 1999a, 1999b et 1999c). La famille de protéines FE65 est constituée de trois membres appelés FE65, COFE65/FE65L1 et FE65L2. La famille de protéines X11 est également constituée de trois membres appelés X11α, X11β et X11γ. Ces deux familles de protéines ont des effets opposés sur la régulation de la production du peptide β-amyloïde. Nous avons montré, ainsi qu'un autre laboratoire, que la sur-expression de FE65 induit une augmentation de la production du peptide Aβ (Mercken *et al*., 1998 ; Sabo *et al*., 1999), alors que la sur-expression de X11 induit une diminution de la production du peptide Aβ (Borg *et al*., 1998 ; Sastre *et al.,* 1998).

L'analyse de la structure primaire de FE65 indique que cette protéine joue vraisemblablement le rôle d'adaptateur. En effet, FE65 contient trois domaines protéiques impliqués dans des interactions protéine-protéine : un domaine WW dans la moitié amino-terminale et deux domaines PTB (PhosphoTyrosine Binding domain), appelés PTB1 et PTB2, dans la moitié carboxy-terminale. La construction de délétions a montré que le domaine PTB2 de FE65 est impliqué dans l'interaction avec le domaine cytoplasmique de l'APP. Le domaine WW interagit avec au moins cinq protéines dont deux ont été identifiées comme étant la protéine Mena (Mammalian homologue of Enabled) (Ermekova *et al*., 1997). De plus, le domaine PTB1 de FE65 interagit avec le facteur de transcription CP2/LSF/LBP1 (Zambrano *et al*., 1997) et avec le récepteur LRP (LDL receptor-Related Protein) (Trommsdorff *et al*., 1998). Le rôle de ces protéines dans la fonction physiologique de FE65 n'est pas connu à ce jour.

L'élucidation du rôle exact de la protéine FE65 dans le processus de production du peptide β-amyloïde constitue donc un enjeu majeur pour la compréhension et l'approche thérapeutique de la maladie d'Alzheimer et plus généralement des maladies neurodégénératives.

La présente invention réside dans l'identification de partenaires de la protéine FE65, interagissant avec cette protéine dans des conditions physiologiques. Ces partenaires représentent de nouvelles cibles pharmacologique pour la fabrication ou la recherche de composés capables de moduler l'activité de FE65, notamment son activité sur la production du peptide β-amyloïde. Ces protéines, les anticorps, les acides nucléiques correspondant ainsi que les sondes ou amorces spécifiques, sont également utilisables pour la détection ou le dosage des protéines dans des échantillons biologiques, notamment des échantillons de tissu nerveux. Ces protéines ou acides nucléiques sont également utilisables dans des approches thérapeutiques, pour moduler l'activité de FE65, ainsi que tout composé selon l'invention, capables de moduler l'interaction entre FE65 et les polypeptides de l'invention.

La présente invention résulte plus particulièrement de la mise en évidence par la demanderesse de deux protéines humaines interagissant avec le domaine PTB1 de FE65 (représenté sur la séquence SEQ ID N° :1 et 2). Ainsi, la présente invention montre que la région centrale de la protéine hnRNPL interagit avec le domaine PTB1 de FE65. Elle décrit également l'identification d'une nouvelle protéine, appelée FEBP1 (FE65 Binding PTB1 domain protein), capable d'interagir avec le domaine PTB1 de FE65.

La présente invention résulte également de l'identification et de la caractérisation de régions particulières des protéines hnRNPL et FEBP1 ci-dessus, impliquées dans la modulation de la fonction de la protéine FE65. La mise en évidence de l'existence de ces protéines et de régions impliquées dans leur fonction permet notamment de préparer de nouveaux composés et/ou compositions utilisables comme agents pharmaceutiques et de développer des méthodes industrielles de criblage de tels composés.

Au sens de la présente invention, la dénomination des protéines hnRNPL et FEBP1 couvre les protéines en elles-mêmes ainsi que toutes leurs formes homologues. Par forme homologue on entend désigner toutes protéines équivalentes à la protéine considérée, d'origine cellulaire diverse et notamment issue de cellules d'origine humaine, ou d'autres organismes, et possédant une activité de même type. De tels homologues comprennent également les variants naturels des protéines indiquées, notamment les variants polymorphiques ou d'épissage. Les protéines (ou polypeptides) homologues peuvent être obtenues par exemple par des expériences d'hybridation entre les acides nucléiques codant. Au sens de l'invention, il suffit qu'une séquence de ce type présente un pourcentage d'identité significatif pour conduire à un comportement physiologique assimilable à ceux des protéines hnRNPL et/ou FEBP1 tels que revendiqués.

La présente invention a pour objet un polypeptide présentant la séquence SEQ ID N° 9.

Un autre objet de l'invention réside dans des anticorps ou fragments d'anticorps polyclonaux ou monoclonaux dirigés contre la protéine présentant la séquence SEQ ID N° 9.

De tels anticorps peuvent être générés par des méthodes connues de l'homme du métier. En particulier, ces anticorps peuvent être préparés par immunisation d'un animal contre un peptide de l'invention, par prélèvement du sang, et isolement des anticorps. Ces anticorps peuvent également être générés par préparation d'hybridomes selon les techniques connues de l'homme de l'art.

Plus préférentiellement, les anticorps ou fragments d'anticorps de l'invention présentent la capacité de moduler au moins partiellement l'interaction des composés peptidiques définis ci-avant ou des protéines hnRNPL et/ou FEBP1 avec le domaine PTB1 de FE65, et peuvent être utilisés pour moduler l'activité de FE65.

Par ailleurs, ces anticorps peuvent également être utilisés pour détecter et/ou doser l'expression des peptides revendiqués dans des échantillons biologiques, et de ce fait, pour renseigner sur leur état d'activation.

Les fragments ou dérivés d'anticorps sont par exemple des fragments Fab, Fab'2, des anticorps simple-chaîne (ScFv), etc. Il s'agit notamment de tout fragment ou dérivé conservant la spécificité antigénique des anticorps dont ils sont issus.

Ces anticorps (ou fragments ou dérivés) sont plus préférentiellement capables de lier un épitope présent dans la séquence comprise entre les résidus 1 et 349 de SEQ ID N° :7 ou entre les résidus 1 et 337 de SEQ ID N° 6.

La présente invention a également pour objet tout acide nucléique codant pour un composé peptidique selon l'invention. Il peut s'agir en particulier d'une séquence comprenant tout ou partie des séquences présentées en SEQ ID N° :8 ou de leurs dérivés. Par séquence dérivée, on entend au sens de la présente invention toute séquence hybridant avec les séquences présentées en SEQ ID N° :8, ou avec un fragment de celles-ci, codant pour un peptide selon l'invention, ainsi que les séquences résultant de ces dernières par dégénérescence du code génétique. Les différentes séquences nucléotidiques de l'invention peuvent être d'origine artificielle ou non. Il peut s'agir de séquences génomiques, d'ADNc, d'ARN, de séquences hybrides ou de séquences synthétiques ou semi-synthétiques. Ces séquences peuvent être obtenues soit par criblage de banques d'ADN (banque d'ADNc, banque d'ADN génomique), soit par synthèse chimique, soit par des méthodes mixtes incluant la modification chimique ou enzymatique de séquences obtenues par criblage de banques. L'hybridation mentionnée ci-dessus est préférentiellement réalisée en condition de stringence élevée, et notamment à une température de 50°C pendant 1 heure dans une solution contenant 8,823g/l de trisodium citrate-2H2O, 17,532g/l de chlorure de sodium et 1 % de dodecyl sulfate de sodium, ou encore dans les conditions décrites par Sambrook et al (1989, pages 9.52-9.55).

Un acide nucléique particulier au sens de l'invention code pour un polypeptide comprenant la séquence SEQ ID N° :9 ou un fragment ou dérivé de celle-ci, notamment pour la protéine FEBP1 humaine. Il s'agit avantageusement d'un acide nucléique comprenant la séquence nucléique SEQ ID N° :8.

Les acides nucléiques selon l'invention peuvent être utilisés pour la production des composés peptidiques de l'invention. La présente demande concerne donc également un procédé de préparation d'un composé peptidique selon lequel on cultive une cellule contenant un acide nucléique selon l'invention, dans des conditions d'expression dudit acide nucléique et on récupère le composé peptidique produit. Dans ce cas, la partie codant pour ledit polypeptide est généralement placée sous le contrôle de signaux permettant son expression dans un hôte cellulaire. Le choix de ces signaux (promoteurs, terminateurs, séquence "leader" de sécrétion, etc.) peut varier en fonction de l'hôte cellulaire utilisé. Par ailleurs, les acides nucléiques de l'invention peuvent faire partie d'un vecteur qui peut être à réplication autonome ou intégratif. Plus particulièrement, des vecteurs à réplication autonome peuvent être préparés en utilisant des séquences à réplication autonome chez l'hôte choisi. S'agissant des vecteurs intégratifs, ceux-ci peuvent être préparés par exemple en utilisant des séquences homologues à certaines régions du génome de l'hôte, permettant, par recombinaison homologue, l'intégration du vecteur. Il peut s'agir d'un vecteur de type plasmidique, épisomique, chromosomique, viral, etc.

Les hôtes cellulaires utilisables pour la production des peptides de l'invention par voie recombinante sont aussi bien des hôtes eucaryotes que procaryotes. Parmi les hôtes eucaryotes qui conviennent, on peut citer les cellules animales, les levures, ou les champignons. En particulier, s'agissant de levures, on peut citer les levures du genre *Saccharomyces*, *Kluyveromyces*, *Pichia*, *Schwanniomyces*, ou *Hansenula.* S'agissant de cellules animales, on peut citer les cellules COS, CHO, C127, PC 12 etc. Parmi les champignons, on peut citer plus particulièrement *Aspergillus* ssp. ou *Trichoderma* ssp. Comme hôtes procaryotes, on préfère utiliser les bactéries suivantes *E*. *coli*, *Bacillus*, ou *Streptomyces.*

Les acides nucléiques selon l'invention peuvent également servir à la réalisation d'oligonucléotides antisens ou d'antisens génétiques utilisables comme agents pharmaceutiques ou diagnostiques. Les séquences antisens sont des oligonucléotides de petite taille, complémentaires du brin codant d'un gène donné, et de ce fait sont capables d'hybrider spécifiquement avec l'ARNm transcrit, inhibant sa traduction en protéine. L'invention a ainsi pour objet les séquences antisens capables d'inhiber au moins partiellement l'interaction des protéines hnRNPL et/ou FEBP 1 sur le domaine PTB1 de FE65. De telles séquences peuvent être constituées par tout ou partie des séquences nucléotidiques définies ci-dessus. Il s'agit généralement de séquences ou de fragments de séquences complémentaires de séquences codant pour des peptides interagissant avec le domaine PTB 1 de FE65. De tels oligonucléotides peuvent être obtenus par fragmentation, ou par synthèse chimique, etc.

Les séquences nucléiques peuvent aussi être utilisées dans le cadre de thérapies, pour le trànsfert et l'expression *in vivo* de séquences antisens ou de peptides capables de moduler l'interaction des protéines hnRNPL et/ou FEBP1 avec le domaine PTB1 de FE65. A cet égard, les séquences peuvent être incorporées dans des vecteurs viraux ou non viraux, permettant leur administration *in vivo* (Kahn, A. *et al*., 1991). A titre de vecteurs viraux conformes à l'invention on peut tout particulièrement citer les vecteurs de type adénovirus, rétrovirus, virus associé à l'adénovirus (AAV) ou virus de l'herpès. La présente demande a également pour objet des virus recombinants défectifs comprenant un acide nucléique codant pour un (poly)peptide selon l'invention.

L'invention permet également la réalisation de sondes nucléotidiques, synthétiques ou non, capables de s'hybrider avec les acides nucléiques définis ci-dessus ou avec leur brin complémentaire. De telles sondes peuvent être utilisées *in vitro* comme outil de diagnostic, pour la détection de l'expression ou surexpression des protéines hnRNPL et/ou FEBP1, ou encore pour la mise en évidence d'anomalies génétiques (mauvais épissage, polymorphismes, mutations ponctuelles, etc.). Ces sondes peuvent également être utilisées pour la mise en évidence et l'isolement de séquences d'acides nucléiques homologues codant pour des peptides tels que définis précédemment, à partir d'autres sources cellulaires et préférentiellement de cellules d'origines humaines. Les sondes de l'invention comportent généralement au moins 10 bases, et elles peuvent par exemple comporter jusqu'à l'intégralité d'une des séquences précitées ou de leur brin complémentaire. Préférentiellement, ces sondes sont marquées préalablement à leur utilisation. Pour cela, différentes techniques connues de l'homme du métier peuvent être employées (marquage radioactif, fluorescent, enzymatique, chimique, etc.).

L'invention a encore pour objet toute composition pharmaceutique comprenant comme principe actif au moins un composé tel que défini ci-dessus, notamment un composé peptidique.

Elle a notamment pour objet toute composition pharmaceutique comprenant comme principe actif au moins un anticorps et/ou un fragment d'anticorps tel que défini ci-dessus, ainsi que toute composition pharmaceutique comprenant comme principe actif au moins un acide nucléique ou un vecteur tel que défini ci-dessus.

Elle a également pour objet toute composition pharmaceutique comprenant comme principe actif une molécule chimique capable d'augmenter ou de diminuer l'interaction entre les protéines hnRNPL et/ou FEBP1 et la protéine FE65.

Par ailleurs, elle a aussi pour objet les compositions pharmaceutiques dans lesquelles les peptides, anticorps, molécules chimiques et séquences nucléotidiques définis ci-dessus sont associés entre-eux ou avec d'autres principes actifs.

Les compositions pharmaceutiques selon l'invention peuvent être utilisées pour moduler l'activité des protéines hnRNPL et/ou FEBP1 et de ce fait modifier la fonction de l'APP, son transport intracellulaire, sa maturation et sa conversion en peptide β-amyloïde. Plus particulièrement, ces compositions pharmaceutiques sont destinées à moduler l'interaction entre les protéines hnRNPL ou FEBP1 et la protéine FE65. Il s'agit plus préférentiellement de compositions pharmaceutiques destinées au traitement de maladies neurodégénératives comme par exemple la maladie d'Alzheimer. Les compositions (ou composés) de l'invention sont plus particulièrement destinés à inhiber, au moins partiellement, l'interaction entre la protéine FE65 et la protéine hnRNPL et/ou FEBP1.

L'invention a encore pour objet l'utilisation des molécules décrites ci-dessus pour moduler l'activité de la protéine FE65 ou le typage de maladies neurodégénératives. En particulier, l'invention concerne l'utilisation de ces molécules pour moduler au moins partiellement l'activité du domaine PTB1 de FE65.

L'invention concerne également un procédé pour le criblage ou la caractérisation de molécules actives sur la fonction de la protéine FE65, comprenant la sélection de molécules capables de lier la séquence SEQ ID N° :7 ou la séquence SEQ ID N° :9, ou un fragment (ou dérivé) de celles-ci. Le procédé comprend avantageusement la mise en contact, in vitro, de la ou des molécules test avec un polypeptide comprenant la séquence SEQ ID N° :7 ou la séquence SEQ ID N° :9, ou un fragment (ou dérivé) de celles-ci, et la sélection de molécules capables de lier la séquence SEQ ID N° :7 (notamment la région comprise entre les résidus 1 et 349) ou la séquence SEQ ID N° :9 (notamment la région comprise entre les résidus 1 et 337). Les molécules testées peuvent être de nature variée (peptide, nucléique, lipide, sucre, etc., ou des mélanges de telles molécules, par exemple des bibliothèques combinatoires, etc.). Comme indiqué ci-avant, les molécules ainsi identifiées peuvent être utilisées pour moduler l'activité de la protéine FE65, et représentent des agents thérapeutiques potentiels pour le traitement de pathologies neurodégénératives.

### MATERIELS ET TECHNIQUES MIS EN OEUVRE

### 1) Souches de levures utilisées :

La souche L40 du genre *S.cerevisiae* (*Mata*, *his3D200*, *trp1-901, leu2-3,112, ade2*, *LYS2:: (lexAop)4-HIS3, URA3::(lexAop)8-LacZ, GAL4, GAL80*) a été utilisée comme outil de criblage de la banque de fusion de cerveau par le système Deux Hybrides. Cette souche permet la mise en évidence d'interaction protéine-protéine quand l'un des partenaires protéiques est fusionné à la protéine LexA (Vojtek *et al*., 1993). Elle a été cultivée sur le milieu de culture suivant :

Milieu YNB minimum :
- Yeast Nitrogen Base (sans acides aminés) (6,7g/l) (Difco)
- Glucose (20g/l) (Merck)

Ce milieu peut être rendu solide par addition de 20g/l d'agar (Difco).

Pour permettre la croissance des levures auxotrophes sur ce milieu, il est nécessaire d'y ajouter les acides aminés ou les bases azotées, pour lesquelles elles sont dépendantes à 50mg/ml. 100 µg/ml d'ampicilline sont ajoutés au milieu afin d'éviter les contaminations bactériennes.

### 2) Souches de bactérie utilisées :

La souche TG1 d'Escherichia coli, de génotype supE, hsdΔ5, thi, Δ(lac-proAB), F'[tra D36 pro A⁺B⁺ lacI^{q} lacZΔM15], a été employée pour la construction de plasmides, et pour l'amplification et l'isolement de plasmides. Elle a été cultivée sur le milieu suivant:
Milieu LB :
   - NaCL(5g/l) (Sigma)
   - Bactotryptone (10g/l)(Difco)
   - Extrait de Levure (5g/l)(Difco)

Ce milieu peut être rendu solide par addition de 20g/l d'agar (Difco).

De l'ampicilline, à 100 µg/ml, a été utilisée pour sélectionner les bactéries ayant reçu les plasmides portants comme marqueur le gène de résistance à cet antibiotique.

### 3) Plasmides mis en oeuvre :

Le vecteur pGAD10, fourni par Clontech® permet l'expression chez la levure de protéines de fusion entre le domaine transactivateur de GAL4 et une protéine codée par l'ADNc provenant d'une banque de cerveau.

Le vecteur pLex9 (pBTM116) (Bartel *et al*., 1993) permet l'expression chez la levure de protéines de fusion avec la protéine LexA.

Le vecteur pGAD424, (Clontech®), permet l'expression chez la levure de protéines de fusion avec le domaine transactivateur de GAL4.

pLex-FE65PTB1, plasmide pLex9 qui contient la séquence codant pour le domaine PTB1 de la protéine FE65 (acides aminés 395 à 543). Ce plasmide a été utilisé pour le criblage de partenaires protéiques du domaine PTB1 de FE65.

pLex-FE65PTB2, plasmide pLex9 qui contient la séquence codant pour le domaine PTB2 de la protéine FE65 (acides aminés 565 à 698) connue pour interagir avec la région cytoplasmique de l'APP (Précurseur du Peptide β-amyloïde). Ce plasmide a été utilisé pour tester la spécificité d'interaction des protéines hnRNPL et FEBP1 avec les domaines PTB de FE65.

pLex-HaRasVal12, plasmide pLex9 qui contient la séquence codant pour la protéine HaRas mutée en position Val12 connue pour interagir avec la protéine Raf de mammifère (Vojtek *et al*., 1993). Ce plasmide a été utilisé pour tester la spécificité d'interaction des protéines hnRNPL et FEBP1 avec FE65.

pGAD-Raf, plasmide pGAD424 qui contient la séquence codant pour la protéine Raf (Vojtek *et al*., 1993). Ce plasmide a été utilisé pour tester la spécificité d'interaction des protéines hnRNPL et FEBP1 avec FE65.

pGAD-App, plasmide pGAD10 qui contient la séquence codant pour le domaine cytoplasmique de la protéine APP connue pour interagir avec le domaine PTB2 de FE65 (Mercken *et al*., 1998). Ce plasmide a été utilisé pour tester la spécificité d'interaction des protéines hnRNPL et FEBP1 avec FE65.

### 4) Oligonucléotides de synthèse employés :

Les oligonucléotides sont synthétisés sur l'appareil Applied System ABI 394-08. Ils sont décrochés de la matrice de synthèse par de l'ammoniac et précipités deux fois par 10 volumes de n-butanol puis repris dans de l'eau. La quantification est effectuée par mesure de la densité optique.
SEQ ID N°3 : CTTCCCGGGTCCCCCACGGAATACCAAC
SEQ ID N°4 : GGGGTCGACGGCATTACGCCGTTCGGC

Ces oligonucléotides ont permis d'obtenir le fragment PCR correspondant au domaine PTB1 de FE65 (représenté sur la séquence SEQ ID N°1), introduisant les sites XmaI et SalI aux extrémités (soulignés).
SEQ ID N°5 : CCACTACAATGGATGATG

Cet oligonucléotide (GAL4TA) a été utilisé pour séquencer les inserts contenus dans les plasmides de la banque Deux Hybrides d'ADNc de cerveau.

### 5) Préparation des ADN plasmidiques

Les préparations en petite quantité et en grande quantité d'ADN plasmidique ont été effectuées selon les protocoles recommandés par le fabricant Quiagen des kits de purification d'ADN :
- Quiaprep Spin Miniprep kit, ref : 27106
- Quiaprep Plasmid Maxiprep kit, ref : 12163.

### 6) Amplification enzymatique d'ADN par PCR (Polymerase Chain Reaction)

Les réactions de PCR sont effectuées dans un volume final de 50µl en présence de la matrice d'ADN, de dNTP (0,2mM), de tampon PCR (Tris-HCL pH 8,5 10 mM, MgCl₂ 1mM, KCl 5 mM, gélatine 0,01%), de 0,5 µg de chacun des oligonucléotides et de 2,5 UI d'Ampli Taq DNA polymérase (Perkin Elmer) avec ou sans formamide (5%). Le mélange est recouvert de 2 gouttes d'huile de paraffine pour limiter l'évaporation de l'échantillon. L'appareil utilisé est le "Crocodile II" d'Appligene.

Nous avons utilisé une température de dénaturation de la matrice de 90°C, une température d'hybridation de 50°C et une température d'élongation par l'enzyme à 72°C.

### 7) Les ligatures

Toutes les réactions de ligation sont effectuées à +14°C pendant une nuit dans un volume final de 10 µl en présence de 100 à 200 ng de vecteur, 0.5 à 2 µg d'insert, 40 UI d'enzyme T4 DNA ligase (Biolabs) et un tampon de ligation (Tris-HCl 50 mM pH 7,8; MgCl₂ 10 mM; DTT 10 mM; ATP 1 mM).

### 8) Transformation des bactéries :

La transformation des bactéries par un plasmide est effectuée selon le protocole suivant : La totalité du volume de ligation (10µl) est utilisée pour transformer les bactéries TG1 rendues compétentes par la méthode de Chung *et al,* (1988).

### 9) Séparation et l'extraction des ADN :

La séparation et l'extraction des fragments d'ADN sont réalisées suivant Sambrook *et al.* (1989).

### 10) Séquençage fluorescent des ADN plasmidiques

La technique de séquençage utilisée est dérivée de la méthode de Sanger *et al*., (1977) et adaptée pour le séquençage par fluorescence et développée par Applied Biosystems. Le protocole utilisé est celui décrit par les concepteurs du système (Perkin Elmer).

### 11) Préparation de plasmides de la banque de cerveau

Cette préparation a été réalisée suivant les recommandations du fournisseur (Clontech®).

### 12) Transformation de la levure par un plasmide

Les levures sont rendues compétentes par un traitement au LiAC/PEG d'après la méthode décrite par Gietz *et al*, (1995).

Dans le cas particulier de la transformation de la levure par la banque d'ADNc de cerveau, on utilise 250 ml à 10⁷ cellules/ml d'une culture, en milieu minimum YNB+His+Lys+Ade+Leu, de levure contenant le plasmide pLex-FE65PTB1. Les levures rendues compétentes, selon le protocole cité ci-dessus, sont transformées par 30 µg d'ADNc de la banque de cerveau. Après les étapes de transformation, les levures sont remises en culture dans 250ml YNB+His+Lys+Ade+Leu à 28°C pendant 16 heures puis récoltées par centrifugation pour être étalées sur un milieu YNB+Lys+Ade et incubées 3 jours à 28°C. La détermination de l'efficacité de transformation et du taux d'amplification a été effectuée suivant le protocole Clontech®.

### 13) Extraction de l'ADN (génomique et plasmidique) de levure

3ml d'une culture de levure incubée 16h à 30°C sont centrifugés et repris dans 200µl d'un tampon de lyse (Sorbitol 1M, KH₂PO₄/K₂HPO₄ 0,1M pH7,4, zymolyase 12,5 mg/ml) et incubés 1h à 37°C. Le lysat est ensuite traité selon le protocole recommandé par le fabriquant Quiagen du kit de purification d'ADN, Quiaprep Spin Miniprep kit, ref : 27106.

### 14) Test d'activité de la β-galactosidase

Une feuille de nitrocellulose est préalablement déposée sur la boîte de Pétri contenant les clones de levures individualisés. Cette feuille est ensuite plongée dans de l'azote liquide pendant 30 secondes afin de faire éclater les levures et de libérer ainsi l'activité β-galactosidase. Après décongélation, la feuille de nitrocellulose est déposée, colonies vers le haut, dans une autre boite de Pétri contenant un papier Whatman préalablement imbibé de 1,5ml de solution PBS (Na₂HPO₄ 60mM, NaH₂PO₄ 40mM, KCl 10mM, MgSO₄ 1mM, pH7) contenant 15 µl de X-Gal (5-bromo-4-chloro-3-indoyl-β-D-galactoside) à 40mg/ml de N,N-diméthylformamide. La boîte est ensuite placée dans une étuve à 37°C. Le test est dit positif lorsque les colonies virent au bleu sur la membrane au bout de 6 heures.

### EXEMPLE 1 : CONSTRUCTION D'UN VECTEUR D'EXPRESSION D'UNE PROTEINE DE FUSION ENTRE LE DOMAINE PTB1 DE FE65 ET LA PROTEINE LEXA

Le criblage d'une banque utilisant le système Double-Hybride nécessite que le domaine PTB1 de FE65 (FE65PTB1) soit fusionné à la protéine LexA. L'expression de cette protéine de fusion est réalisée grâce au vecteur pLex9, dans lequel a été introduite, dans le même cadre de lecture que la séquence correspondant à la protéine LexA, la séquence codant pour le domaine PTB1 de FE65 (SEQ ID N°1-2).

Le fragment d'ADN de 448 pb correspondant aux acides aminés 395 à 543 de la protéine FE65 humaine (SEQ ID N° :2) a été obtenu par PCR à partir des oligonucléotides SEQ ID N°3 et SEQ ID N°4 qui nous ont également permis d'introduire les sites XmaI et SalI aux extrémités de la séquence. Le fragment de PCR a été introduit entre les sites XmaI et SalI du multisite de clonage du plasmide plex9 en aval de la séquence correspondant à LexA pour donner le vecteur pLex-FE65PTB 1.

La construction a été vérifiée par séquençage de l'ADN. Cette vérification nous a permis de montrer que ce fragment ne présentait pas de mutations générées au cours de la réaction de PCR et qu'il était fusionné dans la même phase ouverte de lecture que celle du fragment correspondant à LexA.

### EXEMPLE 2 : CRIBLAGE PAR LA TECHNIQUE DU DEUX HYBRIDES D'UNE BANQUE DE FUSION D'ADNc DE CERVEAU

Nous avons utilisé la méthode du Double-Hybride (Fields et Song, 1989). Le criblage d'une banque de fusion permet d'identifier des clones produisant des protéines fusionnées au domaine transactivateur de GAL4 pouvant interagir avec le domaine PTB1 de FE65. Cette interaction permet de reconstituer un transactivateur qui sera capable d'induire l'expression des gènes rapporteurs His3 et *LacZ* dans la souche L40. Pour effectuer ce criblage nous avons choisi une banque de fusion réalisée à partir d'ADNc de cerveau humain (Clontech^{®}).

Lors du criblage il est nécessaire de préserver la probabilité que chaque plasmide indépendant de la banque de fusion soit présent dans au moins une levure en même temps que le plasmide pLex-FE65PTB1. Pour préserver cette probabilité il est important d'avoir une bonne efficacité de transformation de la levure. Pour cela nous avons choisi un protocole de transformation de la levure donnant une efficacité de 10⁵ cellules transformées par µg d'ADN. De plus comme la cotransformation de la levure par deux plasmides différents réduit cette efficacité, nous avons préféré utiliser une levure préalablement transformée par le plasmide pLex-FE65PTB1. Cette souche L40-FE65PTB1 de phénotype His-, Lys-, Ade- Leu- a été transformée par 30 µg d'ADN plasmidique de la banque de fusion. Cette quantité d'ADN nous a permis d'obtenir après estimation 2,8 10⁶ cellules transformées, ce qui correspond à un nombre légèrement supérieur au nombre de plasmides indépendants que constitue la banque. D'après ce résultat nous pouvons penser que la quasi totalité des plasmides de la banque a servi à transformer les levures. La sélection des cellules transformées, capables de reconstituer un transactivateur GAL4 fonctionnel, a été faite sur un milieu YNB+Lys+Ade.

A l'issu de cette sélection 97 clones avec un phénotype His+ ont été obtenus. Sur ces transformants un test d'activité β-galactosidase a été effectué afin de déterminer le nombre de clones exprimant l'autre gène rapporteur, *LacZ.* Sur 97 clones obtenus, 27 présentaient le double phénotype His+, βGal+ montrant ainsi qu'ils expriment des protéines pouvant interagir avec le domaine PTB1 de FE65.

### EXEMPLE 3 : ISOLEMENT DES PLASMIDES DE LA BANQUE DE CERVEAU À PARTIR DES CLONES DE LEVURES SELECTIONNES

Afin d'identifier les protéines pouvant interagir avec le domaine PTB1 de FE65, nous avons extrait les plasmides de la banque de fusion contenus dans les levures sélectionnées lors du criblage Double-Hybride. Pour pouvoir en obtenir en grande quantité, cet isolement nécessite au préalable une transformation d'E.coli par un extrait d'ADN des souches de levures positives. Comme le plasmide de la banque contenu dans cet extrait est un plasmide navette levure/E.coli il peut facilement se répliquer chez la bactérie.

Les ADN plasmidiques des colonies bactériennes obtenues après transformation par des extraits d'ADN de levures ont été analysées par digestion avec des enzymes de restriction et séparation des fragments d'ADN sur gel d'agarose. Nous avons obtenu 6 profils de restriction différents sur les 23 clones analysés dont 2 étaient fortement représentés. Ces résultats ont montré qu'au moins 6 plasmides différents ont été isolés lors de ce criblage, nous nous sommes plus particulièrement intéressés au fragment d'ADN provenant de la banque d'ADNc contenu dans les deux plasmides les plus représentés (8 et 4 fois).

### EXEMPLE 4 : DETERMINATION DE LA SEQUENCE DES INSERTS DES PLASMIDES IDENTIFIES

Le séquençage a été réalisé sur les 2 plasmides les plus représentés à partir de l'oligonucléotide GAL4TA (SEQ ID N°5) complémentaire de la région de GAL4TA à proximité du site d'insertion de la banque de cDNA de cerveau, à 52 pdb du site EcoRI.

La comparaison de la séquence du premier plasmide sélectionné avec les séquences contenues dans les banques de données GENBank et EMBL (European Molecular Biology Lab) a montré que la séquence de l'ADNc présent dans ce premier plasmide présente plus de 99% d'identité au niveau nucléotidique avec le gène humain codant pour la protéine hnRNPL ayant le numéro d'accession : NP_001524/g4557645. La séquence de ce gène que nous avons cloné par le système Deux Hybrides commence au nucléotide 346 correspondant au 116^{ème} acide aminé et se termine au nucléotide 1392 correspondant au 464^{ème} acide aminé situé à 58 acides aminés de la fin de la protéine hnRNPL (SEQ ID N° :6 et 7). Ce résultat montre que le domaine d'interaction de hnRNPL avec la protéine FE65 humaine est contenu dans la région centrale de hnRNPL. Cette région contient une séquence de type NPXY qui est connue pour être le site consensus de fixation des domaines PTB (Borg *et al*., 1996). La séquence de hnRNPL (SEQ ID N°6-7) que nous avons clonée diffère de la séquence publiée (Pinol-Roma *et al*, 1989) par la substitution d'une Guanine en Thymidine en position 748 conduisant au changement d'une Glycine en Cystéine au niveau du 250^{ème} acide aminé de la séquence SEQ ID N° :6, correspondant au nucléotide 1093 et à l'acide aminé 365 de la protéine hnRNPL entière.

La comparaison de la séquence du deuxième plasmide sélectionné avec les séquences contenues dans les banques de données GENBank et EMBL (European Molecular Biology Lab) a montré que la séquence de l'ADNc contenue dans ce plasmide ne présente aucune homologie significative avec les séquences contenues dans ces banques de données.

Une homologie significative a été trouvée avec la protéine de séquence SEQ ID N°4 de la demande WO99/26961. Cependant la protéine de séquence SEQ ID n°9 objet de la présente demande se distingue de la protéine de séquence SEQ ID N°4 de la demande WO99/26961. En particulier la protéine de séquence SEQ ID n°9 est plus courte de 42 acides aminés. Sur le reste de la séquence il existe une certaine homologie mais aussi des différences notables. En particulier les acides aminés en positions 1, 48, 61, 305 sont différents des acides aminés aux positions corespondantes sur la partie présentant une certaine homologie de la protéine de séquence SEQ ID N°4 de la demande WO99/26961.

Cette séquence (SEQ ID N°8-9) de 1275 nucléotides, présente un codon de terminaison en position 1012, et code pour un peptide de 337 acides aminés. La protéine correspondant à cette séquence a été nommée FEBP1 pour FE65 Binding PTB1 domain protein.

### EXEMPLE 5 : ANALYSE DE LA SPECIFICITE D'INTERACTION ENTRE LES DOMAINES PTB DE FE65 ET LES PROTEINES hnRNPL ET FEBP1

Afin de déterminer la spécificité de l'interaction entre les domaines PTB1 et PTB2 de la protéine FE65 humaine et les protéines hnRNPL et FEBP1, nous avons réalisé un test d'interaction par la méthode Deux Hybrides en utilisant le plasmide pLex-FE65PTB2 codant pour le domaine PTB2 de FE65 fusionné à la protéine LexA, à la place du plasmide pLex-FE65PTB1. L'absence d'interaction entre le domaine PTB2 et ces deux protéines permettrait de monter une spécificité d'interaction avec le domaine PTB1 de FE65.

Pour réaliser ce test nous avons transformé la souche L40 par les plasmides isolés lors du criblage de la banque d'ADNc de cerveau et par le plasmide pLex-FE65PTB2. Des contrôles de spécificité d'interaction ont également été effectués en transformant cette souche par différents plasmides comme indiqué dans le tableau N°1. Un test d'activité βGal sur les cellules transformées par les différents plasmides a été effectué afin de détecter une interaction protéine-protéine. L'ensemble des combinaisons de plasmides, et donc des interactions, testés dans le système Double-Hybride sont reportés dans le Tableau N°1. Les combinaisons de plasmides et le type de vecteur correspondant (pLex ou pGAD) sont indiqués dans les colonnes "Combinaisons de Plasmides". Les signes + et - dans la colonne "Interaction" correspondent aux résultats du test βGal et indiquent respectivement la détection ou la non détection d'interaction protéine-protéine.

D'après le résultat du test (Cf. Tableau N°1), seules les levures transformées par les deux plasmides isolés de la banque d'ADNc de cerveau et par le plasmide pLex-FE65PTB1 présentaient une activité βGal+ montrant ainsi que parmi les deux domaines PTB de FE65, seul le domaine PTB1 interagit avec la partie centrale de hnRNPL ou le fragment de la protéine FEBP1. Le domaine PTB1 de FE65 semble interagir de manière spécifique avec hnRNPL et FEBP1 puisque nous n'avons pas pu montrer d'interactions avec la protéine HaRasVal12 ou le domaine C-terminal de l'APP par la technique du Deux Hybrides.

### REFERENCES BIBLIOGRAPHIQUES

Bartel, P.L., C.-T. Chien, R. Stenglanz and S. Fields. 1993 D.A Hartley Ed, Oxford University press :153.
Borg, J.-P., J. Ooi, E. Levy and B. Margolis. 1996. The Phosphotyrosine Interaction Domains of X11 and FE65 Bind to Distinct Sites on the YENPTY Motif of Amyloid Precursor Protein. Mol. Cell. Biol. 16 : 6229-6241.
Borg, J.-P., Y. Yang, M. De Taddéo-Borg, B. Margolis and R.S. Turner. 1998. The X11a Protein Slows Cellular Amyloid Precursor Protein Processing and Reduces Aβ40 and Aβ42 Secretion. J. Biol. Chem. 273 : 14761-14766.
Bressler, S. L., M.D. Gray, B.L. Sopher, Q. Hu, M.G. Hearn, D.G. Pham, M.B. Dinulos, K.-I. Fukuchi, S.S. Sisodia, M.A. Miller, C.M. Disteche and G.M. Martin. 1996. cDNA cloning and chromosome mapping of the human Fe65 gene : interaction of the conserved cytoplasmic domains of the human β-amyloid precursor protein and its homologues with the mouse Fe65 protein. Hum. Mol. Genet. 5 : 1589-1598.
Chung, C.T., E.L. Suzann, and R.H. Miller. 1989. One-step preparation of competent Escherichia coli : transformation and storage of bacterial cells in the same solution. Proc. Natl. Acad. Sci. USA, 86 : 2172-2175.
Duilio, A., R. Faraonio, G. Minopoli, N. Zambrano and T. Russo. 1998. Fe65L2 : a new member of the Fe65 protein family interacting with the intracellular domain of the Alzheimer's β-amyloid precursor protein. Biochem. J. 330 : 513-519.
Ermekova, K.S., N. Zambrano, H. Linn, G. Minopoli, F. Gertler, T. Russo and M. Sudol. 1997. The WW Domain of Neural Protein FE65 Interacts with Proline-rich Motifs in Mena, the Mammalian Homolog of Drosophila Enabled. J. Biol. Chem. 272 : 32869-32877.
Fields, S. and O. Song. 1989. A novel genetic system to detect protein-protein interactions. Nature. 340 : 245-246
Fiore, F., N. Zambrano, G. Minopoli, V. Donini, A. Duilio and T. Russo. 1995. The Regions of the Fe65 Protein Homologous to the Phosphotyrosine Interaction/Phosphotyrosine Binding Domain of Shc Bind the Intracellular Domain of the Alzheimer's Amyloid Precursor Protein. J. Biol. Chem. 270 : 30853-30856.
Gietz, R.D., R. H. Schiestl, A. R. Willems, and R. A. Woods. 1995 Studies on the transformation of intact yeast cells by LiAC/SS-DNA/PEG procedure. Yeast, 11 : 355-360.
Golde, T.E., S. Estus, L.H. Younkin, D.J. Selkoe, and S.G. Younkin. 1992. Processing of the amyloid protein precursor to potentially amyloidogenic derivatives. Science 255 : 728-730.
Guénette, S.Y., J. Chen, P.D. Jondro and R.E. Tanzi. 1996. Association of a novel human FE65-like protein with the cytoplasmic domain of the b-amyloid precursor protein. Proc. Natl. Acad. Sci. U.S.A. 93 : 10832-10837.
Guénette, S.Y., J. Chen, A. Ferland, C. Haass, A. Capell and R.E. Tanzi. 1999. HFE65L Influences Amyloid Precursor Protein Maturation and Secretion. J. Neurochem. 73 : 985-993.
Koo, E.H., and S.L. Squazzo. 1994. Evidence that production and release of amyloid beta-protein involves the endocytic pathway. J. Biol. Chem. 269: 17386-17389.
Kahn, A. 1991 Thérapie génique : espoirs et limites. Médecine et Sciences. 7 : 705-714.
McLoughlin, D.M. and C.C.J. Miller. 1996. The intracellular cytoplasmic domain of the Alzheimer's disease amyloid precursor proteins in the yeast two-hybrid system. FEBS Lett. 397 : 197-200.
Mercken, L., M. Bock, J. Ménager, X. Franco, M.-F. Paul, L. Pradier and A. Fournier. 1998. FE65 and COFE65 : two proteins interacting with the cytoplasmic domain of APP. Neurobiol. Aging 19 : S37.
Perez, R.G., S. Soriano, J.D. Hayes, B. Ostaszewski, W. Xia,D.J. Selkoe, X. Chen, G.B. Stokin, and E.H. Koo. 1999. Mutagenesis identifies new signals for beta-amyloid precursor protein endocytosis, turnover, and the generation of secreted fragments, including Abeta42. J. Biol. Chem. 274 : 18851-18856.
Pinol-Roma, S., M.S Swanson, J.G Gall, and G. Dreyfuss. 1989. A novel heterogeneous nuclear RNP protein with a unique distribution on nascent transcripts. J. Cell Biol. 109 : 2575-2587.
Sabo, S.L., L.M. Lanier, A.F. Ikin, O. Khorkova, S. Sahasrabudhe, P. Greengard and J.D. Buxbaum. 1999. Regulation of the β-Amyloid Secretion by FE65, an Amyloid Protein Precursor-binding Protein. J. Biol. Chem. 274 : 7952-7957.
Sambrook, J., E.F. Fritsch, and T. Maniatis. 1989. Molecular Cloning, A laboratory manual. Second Edition, Tome I, II, III. Cold Spring Harbor Laboratory Press.
Sanger, F., S. Nicklen, and A.R. Coulson. 1977. DNA sequencing with Chain Terminating Inhibitors. Proc. Natl. Acad. Sci. USA, 74 : 5463-5467.
Sastre, M., R.S. Turner, and E. Levy. 1998. X11 Interaction with β-Amyloid Precursor Protein Modulates Its Cellular Stabilization and Reduces Amyloid β-Protein Secretion. J. Biol. Chem. 273 : 22351-22357.
Tanahashi, H. and T. Tabira. 1999a. X11L2, a new member of the X11 protein family, interacts with Alzheimer's beta-amyloid precursor protein. Biochem. Biophys. Res. Commun. 255 : 663-667.
Tanahashi, H., and T. Tabira. 1999b. Genome Structure and Chromosomal Mapping of the Gene for Fe65L2 Interacting with Alzheimer's β-Amyloid Precursor Protein. Biochem. Biophys. Res. Commun. 258 : 385-389.
Tanahashi, H., and T. Tabira. 1999c. Molecular cloning of human Fe65L2 and its interaction with the Alzheimer's beta-amyloid precursor protein. Neurosc. Lett. 261 : 143-146.
Trommsdorff, M., J.-P. Borg, B. Margolis and J. Herz. 1998. Interaction of Cytosolic Adaptor Proteins with Neuronal Apolipoprotein E Receptors and the Amyloid Precursor Protein. J. Biol. Chem. 273 : 33556-33560.
Vojtek, A.B., S.M. Hollenberg, and J.A.Cooper. 1993. Mammalian Ras interacts directly with the serine/threonine kinase Raf. Cell 74 : 205-214
Zambrano, N., J.D. Buxbaum, G. Minopoli, F. Fiore, P. De Candia, S. De Renzis, R. Faraonio, S. Sabo, J. Cheetham, M. Sudol and T. Russo. 1997. Interaction of the Phosphotyrosine Interaction/Phosphotyrosine Binding-related Domains of Fe65 with Wild-type and Mutant Alzheimer's β-Amyloid Precursor Proteins. J. Biol. Chem. 272 :6399-6405.
Yankner, B.A. 1996. Mechanisms of neuronal degeneration in Alzheimer's disease. Neuron 16: 921-932.

**TABLEAU N°1**

| **COMBINAISON DE PLASMIDES** | | **INTERACTION** |
|---|---|---|
| **pLex** | **pGAD** | |
| FE65PTB1 | hnRNPL | + |
| FE65PTB1 | FEBP1 | + |
| FE65PTB1 | APP | - |
| FE65PTB1 | Raf | - |
| FE65PTB2 | hnRNPL | - |
| FE65PTB2 | FEBP1 | - |
| FE65PTB2 | APP | + |
| FE65PTB2 | Raf | - |
| HaRasVal12 | hnRNPL | - |
| HaRasVal12 | FEBP1 | - |
| HaRasVal12 | APP | - |
| HaRasVal12 | Raf | + |

## Revendications

1. Polypeptide présentant la séquence SEQ ID N° : 9.

2. Acide nucléique codant pour le polypeptide selon la revendication 1.

3. Acide nucléique selon la revendication 2, **caractérisé en ce qu'**il comprend la séquence SEQ ID N° : 8.

4. Vecteur comprenant un acide nucléique selon l'une des revendications 2 et 3.

5. Virus recombinant défectif comprenant un acide nucléique selon l'une des revendications 2 à 3.

6. Anticorps ou fragment d'anticorps, **caractérisé en ce qu'**il est dirigé contre le polypeptide selon la revendication 1.

7. Composition pharmaceutique comprenant au moins le polypeptide selon la revendications 1 ou un anticorps selon la revendication 6.

8. Composition pharmaceutique comprenant au moins un acide nucléique selon l'une des revendications 2 et 3 ou un vecteur selon l'une des revendications 4 et 5.

9. Composition selon l'une des revendications 7 et 8 destinée à moduler, au moins partiellement, l'interaction entre la protéine FE65 et la protéine hnRNPL ou le polypeptide présentant la séquence SEQ ID N° 9.

10. Composition selon l'une des revendications 7 et 8 destinée au traitement de pathologies neurodégénératives.

11. Procédé pour le criblage ou la caractérisation de molécules actives dans le traitement de pathologies neurodégénératives, comprenant une étape de sélection de molécules capables de lier la séquence SEQ ID N° 7 ou la séquence SEQ ID N° 9, ou un fragment de celles-ci capable d'interagir avec le domaine PTB1 de FE65.

12. Procédé de production d'un polypeptide selon la revendication 1, comprenant la culture d'une cellule contenant un acide nucléique selon l'une des revendications 2 et 3 ou un vecteur ou virus selon l'une des revendications 4 et 5, dans des conditions d'expression dudit acide nucléique, et la récupération du polypeptide produit.

## Claims

1. Polypeptide having the sequence SEQ ID No: 9.

2. Nucleic acid encoding the polypeptide according to Claim 1.

3. Nucleic acid according to Claim 2, **characterized in that** it comprises the sequence SEQ ID No: 8.

4. Vector comprising a nucleic acid according to either of Claims 2 and 3.

5. Defective recombinant virus comprising a nucleic acid according to one of Claims 2 to 3.

6. Antibody or antibody fragment, **characterized in that** it is directed against the polypeptide according to Claim 1.

7. Pharmaceutical composition comprising at least the polypeptide according to Claim 1 or an antibody according to Claim 6.

8. Pharmaceutical composition comprising at least one nucleic acid according to either of Claims 2 and 3 or a vector according to either of Claims 4 and 5.

9. Composition according to either of Claims 7 and 8, intended for modulating, at least partially, the interaction between the protein FE65 and the protein hnRNPL or the polypeptide having the sequence SEQ ID NO. 9.

10. Composition according to either of Claims 7 and 8, intended for the treatment of neurodegenerative pathologies.

11. Method for the screening or characterization of molecules that are active in the treatment of neurodegenerative pathologies, comprising a step for selecting molecules capable of binding the sequence SEQ ID No. 7 or the sequence SEQ ID No. 9, or a fragment thereof capable of interacting with the PTB1 domain of FE65.

12. Method for producing a polypeptide according to Claim 1, comprising culturing a cell containing a nucleic acid according to either of Claims 2 and 3 or a vector or virus according to either of Claims 4 and 5, under conditions for expressing the said nucleic acid, and recovering the polypeptide produced.

## Patentansprüche

1. Polypeptid mit der Sequenz SEQ ID NR.:9.

2. Nukleinsäure, die für das Polypeptid nach Anspruch 1 codiert.

3. Nukleinsäure nach Anspruch 2, **dadurch gekennzeichnet, daß** sie die Sequenz SEQ ID NR.:8 umfaßt.

4. Vektor, der eine Nukleinsäure nach einem der Ansprüche 2 und 3 umfaßt.

5. Rekombinantes defizientes Virus, das eine Nukleinsäure nach einem der Ansprüche 2 bis 3 umfaßt.

6. Antikörper oder Antikörperfragment, **dadurch gekennzeichnet, daß** er/es gegen das Polypeptid nach Anspruch 1 gerichtet ist.

7. Pharmazeutische Zusammensetzung, die mindestens das Polypeptid nach Anspruch 1 oder einen Antikörper nach Anspruch 6 umfaßt.

8. Pharmazeutische Zusammensetzung, die mindestens eine Nukleinsäure nach einem der Ansprüche 2 und 3 oder einen Vektor nach einem der Ansprüche 4 und 5 umfaßt.

9. Zusammensetzung nach einem der Ansprüche 7 und 8 für die zumindest teilweise Modulation der Wechselwirkung zwischen dem Protein FE65 und dem Protein hnRNPL oder dem Polypeptid mit der Sequenz SEQ ID NR.:9.

10. Zusammensetzung nach einem der Ansprüche 7 und 8 für die Behandlung von neurodegenerativen Erkrankungen.

11. Verfahren für die Durchmusterung oder Charakterisierung von Molekülen mit Aktivität in der Behandlung von neurodegenerativen Erkrankungen, umfassend einen Schritt der Selektion von Molekülen, die fähig sind, an die Sequenz SEQ ID NR.:7 oder die Sequenz SEQ ID NR.:9 zu binden, oder Fragment dieser Moleküle, das fähig ist, eine Wechselwirkung mit der PTB1-Domäne von FE65 einzugehen.

12. Verfahren zur Herstellung eines Polypeptids nach Anspruch 1, bei dem man eine Zelle, die eine Nukleinsäure nach einem der Ansprüche 2 und 3 oder einen Vektor bzw. ein Virus nach einem der Ansprüche 4 und 5 enthält, unter Bedingungen kultiviert, unter denen diese Nukleinsäure exprimiert wird, und das erzeugte Polypeptid gewinnt.


### LISTE DE SEQUENCES

<110> Aventis Pharma SA
<120> Partenaires du domaine PTB1 de FE65, préparation et utilisations
<130> PRJ99058 - RPR
<140>
   <141>
<160> 9
<170> PatentIn Ver. 2.1
<210> 1
   <211> 447
   <212> ADN
   <213> Homo sapiens
<220>
   <221> CDS
   <222> (1)..(447)
<400> 1
   ccc cca cgg aat acc aac cca ggg atc aag tgt ttc gcc gtg cgc tcc 48
   Pro Pro Arg Asn Thr Asn Pro Gly Ile Lys Cys Phe Ala Val Arg Ser
   1 5 10 15
   cta ggc tgg gta gag atg acc gag gag gag ctg gcc cct gga cgc agc 96
   Leu Gly Trp Val Glu Met Thr Glu Glu Glu Leu Ala Pro Gly Arg Ser
   20 25 30
   agt gtg gca gtc aac aat tgc atc cgt cag ctc tct tac cac aaa aac 144
   Ser Val Ala Val Asn Asn Cys Ile Arg Gln Leu Ser Tyr His Lys Asn
   35 40 45
   aac ctg cat gac ccc atg tct ggg ggc tgg ggg gaa gga aag gat ctg 192
   Asn Leu His Asp Pro Met Ser Gly Gly Trp Gly Glu Gly Lys Asp Leu
   50 55 60
   cta ctg cag ctg gag gat gag aca cta aag cta gtg gag cca cag agc 240
   Leu Leu Gln Leu Glu Asp Glu Thr Leu Lys Leu Val Glu Pro Gln Ser
   65 70 75 80
   cag gca ctg ctg cac gcc caa ccc atc atc agc atc cgc gtg tgg ggc 288
   Gln Ala Leu Leu His Ala Gln Pro Ile Ile Ser Ile Arg Val Trp Gly
   85 90 95
   gtc ggg cgg gac agt gga agg gac ttt gcc tac gta gct cgt gat aag 336
   Val Gly Arg Asp Ser Gly Arg Asp Phe Ala Tyr Val Ala Arg Asp Lys
   100 105 110
   ctg acc cag atg ctc aag tgc cac gtg ttt cgc tgt gag gca cct gcc 384
   Leu Thr Gln Met Leu Lys Cys His Val Phe Arg Cys Glu Ala Pro Ala
   115 120 125
   aag aac atc gcc acc agc ctg cat gag atc tgc tct aag atc atg gcc 432
   Lys Asn Ile Ala Thr Ser Leu His Glu Ile Cys Ser Lys Ile Met Ala
   130 135 140
   gaa cgg cgt aat gcc 447
   Glu Arg Arg Asn Ala
   145
<210> 2
   <211> 149
   <212> PRT
   <213> Homo sapiens
<400> 2
   Pro Pro Arg Asn Thr Asn Pro Gly Ile Lys Cys Phe Ala Val Arg Ser
    1               5                  10                  15
   Leu Gly Trp Val Glu Met Thr Glu Glu Glu Leu Ala Pro Gly Arg Ser
               20                  25                  30
   Ser Val Ala Val Asn Asn Cys Ile Arg Gln Leu Ser Tyr His Lys Asn
           35                  40                  45
   Asn Leu His Asp Pro Met Ser Gly Gly Trp Gly Glu Gly Lys Asp Leu
       50                  55                  60
   Leu Leu Gln Leu Glu Asp Glu Thr Leu Lys Leu Val Glu Pro Gln Ser
   65                  70                  75                  80
   Gln Ala Leu Leu His Ala Gln Pro Ile Ile Ser Ile Arg Val Trp Gly
                   85                  90                  95
   Val Gly Arg Asp Ser Gly Arg Asp Phe Ala Tyr Val Ala Arg Asp Lys
               100                 105                 110
   Leu Thr Gln Met Leu Lys Cys His Val Phe Arg Cys Glu Ala Pro Ala
           115                 120                 125
   Lys Asn Ile Ala Thr Ser Leu His Glu Ile Cys Ser Lys Ile Met Ala
       130                 135                 140
   Glu Arg Arg Asn Ala
   145
<210> 3
   <211> 28
   <212> ADN
   <213> Séquence artificielle
<220>
   <223> Description de la séquence artificielle:Oligonucleotide
<400> 3
   cttcccgggt cccccacgga ataccaac 28
<210> 4
   <211> 27
   <212> ADN
   <213> Séquence artificielle
<220>
   <223> Description de la séquence artificielle:Oligonucleotide
<400> 4
   ggggtcgacg gcattacgcc gttcggc 27
<210> 5
   <211> 18
   <212> ADN
   <213> Séquence artificielle
<220>
   <223> Description de la séquence artificielle:Oligonucleotide
<400> 5
   ccactacaat ggatgatg 18
<210> 6
   <211> 1047
   <212> ADN
   <213> Homo sapiens
<220>
   <221> CDS
   <222> (1)..(1047)
<400> 6
   gtg ttg ggg gct tgc aac gca gtg aac tac gca gcc gac aac caa ata    48
   Val Leu Gly Ala Cys Asn Ala Val Asn Tyr Ala Ala Asp Asn Gln Ile
     1               5                  10                  15
   tac att gct ggt cac cca gct ttt gtc aac tac tct acc agc cag aag    96
   Tyr Ile Ala Gly His Pro Ala Phe Val Asn Tyr Ser Thr Ser Gln Lys
                20                  25                  30
   atc tcc cgc cct ggg gac tcg gat gac tcc cgg agc gtg aac agt gtg    144
   Ile Ser Arg Pro Gly Asp Ser Asp Asp Ser Arg Ser Val Asn Ser Val
            35                  40                  45
   ctt ctc ttt acc atc ctg aac ccc att tat tcg atc acc acg gat gtt    192
   Leu Leu Phe Thr Ile Leu Asn Pro Ile Tyr Ser Ile Thr Thr Asp Val
        50                  55                  60
   ctt tac act atc tgt aat cct tgt ggc cct gtc cag aga att gtc att    240
   Leu Tyr Thr Ile Cys Asn Pro Cys Gly Pro Val Gln Arg Ile Val Ile
    65                  70                  75                  80
   ttc agg aag aat gga gtt cag gcg atg gtg gaa ttt gac tca gtt caa    288
   Phe Arg Lys Asn Gly Val Gln Ala Met Val Glu Phe Asp Ser Val Gln
                    85                  90                  95
   agt gcc cag cgg gcc aag gcc tct ctc aat ggg gct gat atc tat tct    336
   Ser Ala Gln Arg Ala Lys Ala Ser Leu Asn Gly Ala Asp Ile Tyr Ser
               100                 105                 110
   ggc tgt tgc act ctg aag atc gaa tac gca aag cct aca cgc ttg aat    384
   Gly Cys Cys Thr Leu Lys Ile Glu Tyr Ala Lys Pro Thr Arg Leu Asn
           115                 120                 125
   gtg ttc aag aat gat cag gat act tgg gac tac aca aac ccc aat ctc    432
   Val Phe Lys Asn Asp Gln Asp Thr Trp Asp Tyr Thr Asn Pro Asn Leu
       130                 135                 140
   agt gga caa ggt gac cct ggc agc aac ccc aac aaa cgc cag agg cag    480
   Ser Gly Gln Gly Asp Pro Gly Ser Asn Pro Asn Lys Arg Gln Arg Gln
   145                 150                 155                 160
   ccc cct ctc ctg gga gat cac ccc gca gaa tat gga ggg ccc cac ggt     528
   Pro Pro Leu Leu Gly Asp His Pro Ala Glu Tyr Gly Gly Pro His Gly
                   165                 170                 175
   ggg tac cac agc cat tac cat gat gag ggc tac ggg ccc ccc cca cct     576
   Gly Tyr His Ser His Tyr His Asp Glu Gly Tyr Gly Pro Pro Pro Pro
               180                 185                 190
   cac tac gaa ggg aga agg atg ggt cca cca gtg ggg ggt cac cgt cgg     624
   His Tyr Glu Gly Arg Arg Met Gly Pro Pro Val Gly Gly His Arg Arg
           195                 200                 205
   ggc cca agt cgc tac ggc ccc cag tat ggg cac ccc cca ccc cct ccc     672
   Gly Pro Ser Arg Tyr Gly Pro Gln Tyr Gly His Pro Pro Pro Pro Pro
       210                 215                 220
   cca cca ccc gag tat ggc cct cac gcc gac agc cct gtg ctc atg gtc     720
   Pro Pro Pro Glu Tyr Gly Pro His Ala Asp Ser Pro Val Leu Met Val
   225                 230                 235                 240
   tat ggc ttg gat caa tct aag atg aac tgt gac cga gtc ttc aat gtc     768
   Tyr Gly Leu Asp Gln Ser Lys Met Asn Cys Asp Arg Val Phe Asn Val
                   245                 250                 255
   ttc tgc tta tat ggc aat gtg gag aag gtg aaa ttc atg aaa agc aag     816
   Phe Cys Leu Tyr Gly Asn Val Glu Lys Val Lys Phe Met Lys Ser Lys
               260                 265                 270
   ccg ggg gcc gcc atg gtg gag atg gct gat ggc tac gct gta gac cgg     864
   Pro Gly Ala Ala Met Val Glu Met Ala Asp Gly Tyr Ala Val Asp Arg
           275                 280                 285
   gcc att acc cac ctc aac aac aac ttc atg ttt ggg cag aag ctg aat     912
   Ala Ile Thr His Leu Asn Asn Asn Phe Met Phe Gly Gln Lys Leu Asn
       290                 295                 300
   gtc tgt gtc tcc aag cag cca gcc atc atg cct ggt cag tca tac ggg     960
   Val Cys Val Ser Lys Gln Pro Ala Ile Met Pro Gly Gln Ser Tyr Gly
   305                 310                 315                 320
   ttg gaa gac ggg tct tgc agt tac aaa gac ttc agt gaa tcc cgg aac     1008
   Leu Glu Asp Gly Ser Cys Ser Tyr Lys Asp Phe Ser Glu Ser Arg Asn
                   325                 330                 335
   aat cgg ttc tcc acc cca gag cag gca gcc aag aac cgc                 1047
   Asn Arg Phe Ser Thr Pro Glu Gln Ala Ala Lys Asn Arg
   340 345
<210> 7
   <211> 349
   <212> PRT
   <213> Homo sapiens
<400> 7
   Val Leu Gly Ala Cys Asn Ala Val Asn Tyr Ala Ala Asp Asn Gln Ile
   1               5                   10                  15
   Tyr Ile Ala Gly His Pro Ala Phe Val Asn Tyr Ser Thr Ser Gln Lys
               20                  25                  30
   Ile Ser Arg Pro Gly Asp Ser Asp Asp Ser Arg Ser Val Asn Ser Val
           35                  40                  45
   Leu Leu Phe Thr Ile Leu Asn Pro Ile Tyr Ser Ile Thr Thr Asp Val
       50                  55                  60
   Leu Tyr Thr Ile Cys Asn Pro Cys Gly Pro Val Gln Arg Ile Val Ile
   65                  70                  75                  80
   Phe Arg Lys Asn Gly Val Gln Ala Met Val Glu Phe Asp Ser Val Gln
                   85                  90                  95
   Ser Ala Gln Arg Ala Lys Ala Ser Leu Asn Gly Ala Asp Ile Tyr Ser
               100                 105                 110
   Gly Cys Cys Thr Leu Lys Ile Glu Tyr Ala Lys Pro Thr Arg Leu Asn
           115                 120                 125
   Val Phe Lys Asn Asp Gln Asp Thr Trp Asp Tyr Thr Asn Pro Asn Leu
       130                 135                 140
   Ser Gly Gln Gly Asp Pro Gly Ser Asn Pro Asn Lys Arg Gln Arg Gln
   145                 150                 155                 160
   Pro Pro Leu Leu Gly Asp His Pro Ala Glu Tyr Gly Gly Pro His Gly
                   165                 170                 175
   Gly Tyr His Ser His Tyr His Asp Glu Gly Tyr Gly Pro Pro Pro Pro
               180                 185                 190
   His Tyr Glu Gly Arg Arg Met Gly Pro Pro Val Gly Gly His Arg Arg
           195                 200                 205
   Gly Pro Ser Arg Tyr Gly Pro Gln Tyr Gly His Pro Pro Pro Pro Pro
       210                 215                 220
   Pro Pro Pro Glu Tyr Gly Pro His Ala Asp Ser Pro Val Leu Met Val
   225                 230                 235                 240
   Tyr Gly Leu Asp Gln Ser Lys Met Asn Cys Asp Arg Val Phe Asn Val
                   245                 250                 255
   Phe Cys Leu Tyr Gly Asn Val Glu Lys Val Lys Phe Met Lys Ser Lys
               260                 265                 270
   Pro Gly Ala Ala Met Val Glu Met Ala Asp Gly Tyr Ala Val Asp Arg
           275                 280                 285
   Ala Ile Thr His Leu Asn Asn Asn Phe Met Phe Gly Gln Lys Leu Asn
       290                 295                 300
   Val Cys Val Ser Lys Gln Pro Ala Ile Met Pro Gly Gln Ser Tyr Gly
   305                 310                 315                 320
   Leu Glu Asp Gly Ser Cys Ser Tyr Lys Asp Phe Ser Glu Ser Arg Asn
                   325                 330                 335
   Asn Arg Phe Ser Thr Pro Glu Gln Ala Ala Lys Asn Arg
               340                 345
<210> 8
   <211> 1275
   <212> ADN
   <213> Homo sapiens
<220>
   <221> CDS
   <222> (1)..(1014)
<400> 8
   cgg ggg gat gtg gat gat gct ggg gac tgt tct ggg gcc agg tat aat      48
   Arg Gly Asp Val Asp Asp Ala Gly Asp Cys Ser Gly Ala Arg Tyr Asn
     1               5                  10                  15
   gac tgg tct gat gat gat gat gac agc aat gag agc aag agt ata gta      96
   Asp Trp Ser Asp Asp Asp Asp Asp Ser Asn Glu Ser Lys Ser Ile Val
               20                  25                  30
   tgg tac cca cct tgg gct cgg att ggg act gaa gct gga acc aga gct     144
   Trp Tyr Pro Pro Trp Ala Arg Ile Gly Thr Glu Ala Gly Thr Arg Ala
           35                  40                  45
   agg gcc agg gca agg gcc agg gct acc cgg gca cgt cgg gct gtc cag     192
   Arg Ala Arg Ala Arg Ala Arg Ala Thr Arg Ala Arg Arg Ala Val Gln
       50                  55                  60
   aaa cgg gct tcc ccc aat tca gat gat acc gtt ttg tcc cct caa gag     240
   Lys Arg Ala Ser Pro Asn Ser Asp Asp Thr Val Leu Ser Pro Gln Glu
   65                  70                  75                  80
   cta caa aag gtt ctt tgc ttg gtt gag atg tct gaa aag cct tat att     288
   Leu Gln Lys Val Leu Cys Leu Val Glu Met Ser Glu Lys Pro Tyr Ile
                   85                  90                  95
   ctt gaa gca gct tta att gct ctg ggt aac aat gct gct tat gca ttt     336
   Leu Glu Ala Ala Leu Ile Ala Leu Gly Asn Asn Ala Ala Tyr Ala Phe
               100                 105                 110
   aac aga gat att att cgt gat ctg ggt ggt ctc cca att gtc gca aag     384
   Asn Arg Asp Ile Ile Arg Asp Leu Gly Gly Leu Pro Ile Val Ala Lys
           115                 120                 125
   att ctc aat act cgg gat ccc ata gtt aag gaa aag gct tta att gtc     432
   Ile Leu Asn Thr Arg Asp Pro Ile Val Lys Glu Lys Ala Leu Ile Val
       130                 135                 140
   ctg aat aac ttg agt gtg aat gct gaa aat cag cgc agg ctt aaa gta     480
   Leu Asn Asn Leu Ser Val Asn Ala Glu Asn Gln Arg Arg Leu Lys Val
   145                 150                 155                 160
   tac atg aat caa gtg tgt gat gac aca atc act tct cgc ttg aac tca     528
   Tyr Met Asn Gln Val Cys Asp Asp Thr Ile Thr Ser Arg Leu Asn Ser
                   165                 170                 175
   tct gtg cag ctt gct gga ctg aga ttg ctt aca aat atg act gtt act     576
   Ser Val Gln Leu Ala Gly Leu Arg Leu Leu Thr Asn Met Thr Val Thr
               180                 185                 190
   aat gag tat cag cac atg ctt gct aat tcc att tct gac ttt ttt cgt     624
   Asn Glu Tyr Gln His Met Leu Ala Asn Ser Ile Ser Asp Phe Phe Arg
           195                 200                 205
   tta ttt tca gcg gga aat gaa gaa acc aaa ctt cag gtt ctg aaa ctc     672
   Leu Phe Ser Ala Gly Asn Glu Glu Thr Lys Leu Gln Val Leu Lys Leu
       210                 215                 220
   ctt ttg aat ttg gct gaa aat cca gcc atg act agg gaa ctg ctc agg     720
   Leu Leu Asn Leu Ala Glu Asn Pro Ala Met Thr Arg Glu Leu Leu Arg
   225                 230                 235                 240
   gcc caa gta cca tct tca ctg ggc tcc ctc ttt aat aag aag gag aac    768
   Ala Gln Val Pro Ser Ser Leu Gly Ser Leu Phe Asn Lys Lys Glu Asn
                   245                 250                 255
   aaa gaa gtt att ctt aaa ctt ctg gtc ata ttt gag aac ata aat gat    816
   Lys Glu Val Ile Leu Lys Leu Leu Val Ile Phe Glu Asn Ile Asn Asp
               260                 265                 270
   aat ttc aaa tgg gaa gaa aat gaa cct act cag aat caa ttc ggt gaa    864
   Asn Phe Lys Trp Glu Glu Asn Glu Pro Thr Gln Asn Gln Phe Gly Glu
           275                 280                 285
   ggt tca ctt ttt ttc ttt tta aaa gaa ttt caa gtg tgt gct gat aag    912
   Gly Ser Leu Phe Phe Phe Leu Lys Glu Phe Gln Val Cys Ala Asp Lys
       290                 295                 300
   gnt ctg gga ata gaa agt cac cat gat ttt ttg gtg aaa gta aaa gtt    960
   Xaa Leu Gly Ile Glu Ser His His Asp Phe Leu Val Lys Val Lys Val
   305                 310                 315                 320
   gga aaa ttc atg gcc aaa ctt gct gaa cat atg ttc cca aag agc cag    1008
   Gly Lys Phe Met Ala Lys Leu Ala Glu His Met Phe Pro Lys Ser Gln
                   325                 330                 335
   gaa taa caccttgatt ttgtaattta gaagcaacac acattgtaaa ctattcattt    1064
   Glu
   tctccacctt gtttatatgg taaaggaatc ctttcagctg ccagttttga ataatgaata 1124
   tcatattgta tcatcaatgc tgatatttaa ctgagttggt ctttaggttt aagatggata 1184
   aatgaatatc actacttgtt ctgaaaacat gtttgttgct ttttatctcg ctgcctagat 1244
   tgaaatattt tgctatttct tctggctaaa g                                 1275
<210> 9
   <211> 337
   <212> PRT
   <213> Homo sapiens
<400> 9
   Arg Gly Asp Val Asp Asp Ala Gly Asp Cys Ser Gly Ala Arg Tyr Asn
   1 5 10 15
   Asp Trp Ser Asp Asp Asp Asp Asp Ser Asn Glu Ser Lys Ser Ile Val
   20 25 30
   Trp Tyr Pro Pro Trp Ala Arg Ile Gly Thr Glu Ala Gly Thr Arg Ala
   35 40 45
   Arg Ala Arg Ala Arg Ala Arg Ala Thr Arg Ala Arg Arg Ala Val Gln
   50 55 60
   Lys Arg Ala Ser Pro Asn Ser Asp Asp Thr Val Leu Ser Pro Gln Glu
   65 70 75 80
   Leu Gln Lys Val Leu Cys Leu Val Glu Met Ser Glu Lys Pro Tyr Ile
   85 90 95
   Leu Glu Ala Ala Leu Ile Ala Leu Gly Asn Asn Ala Ala Tyr Ala Phe
   100 105 110
   Asn Arg Asp Ile Ile Arg Asp Leu Gly Gly Leu Pro Ile Val Ala Lys
   115 120 125
   Ile Leu Asn Thr Arg Asp Pro Ile Val Lys Glu Lys Ala Leu Ile Val
   130 135 140
   Leu Asn Asn Leu Ser Val Asn Ala Glu Asn Gln Arg Arg Leu Lys Val
   145 150 155 160
   Tyr Met Asn Gln Val Cys Asp Asp Thr Ile Thr Ser Arg Leu Asn Ser
   165 170 175
   Ser Val Gln Leu Ala Gly Leu Arg Leu Leu Thr Asn Met Thr Val Thr
   180 185 190
   Asn Glu Tyr Gln His Met Leu Ala Asn Ser Ile Ser Asp Phe Phe Arg
   195 200 205
   Leu Phe Ser Ala Gly Asn Glu Glu Thr Lys Leu Gln Val Leu Lys Leu
   210 215 220
   Leu Leu Asn Leu Ala Glu Asn Pro Ala Met Thr Arg Glu Leu Leu Arg
   225 230 235 240
   Ala Gln Val Pro Ser Ser Leu Gly Ser Leu Phe Asn Lys Lys Glu Asn
   245 250 255
   Lys Glu Val Ile Leu Lys Leu Leu Val Ile Phe Glu Asn Ile Asn Asp
   260 265 270
   Asn Phe Lys Trp Glu Glu Asn Glu Pro Thr Gln Asn Gln Phe Gly Glu
   275 280 285
   Gly Ser Leu Phe Phe Phe Leu Lys Glu Phe Gln Val Cys Ala Asp Lys
   290 295 300
   Xaa Leu Gly Ile Glu Ser His His Asp Phe Leu Val Lys Val Lys Val
   305 310 315 320
   Gly Lys Phe Met Ala Lys Leu Ala Glu His Met Phe Pro Lys Ser Gln
   325 330 335
   Glu